# EUROPEAN PATENT SPECIFICATION

(11) **EP 1 481 687 B1**
(45) Date of publication and mention of the grant of the patent: **15.08.2007**
(21) Application number: 04356079.6
(22) Date of filing: 25.05.2004
(51) Int. Cl.: A61K 38/19, A61P 35/00, A61K 47/48

(54) **Use of multimeric ligands of the TNF family with reduced toxicity for treating cell proliferative diseases**
Verwendung von TNF-Liganden Multimere mit reduzierter Toxicität zur Behandlung von proliferativen Krankheiten
Utilisation de multimères de ligands de la famille du TNF à toxicité réduite dans le traitement de maladies prolifératives

(30) Priority: 26.05.2003 EP 03291247
(43) Date of publication of application: 01.12.2004
(73) Proprietor: APOXIS SA, 1004 Lausanne (CH)
(72) Inventor: Rosat, Jean-Pierre, 1092 Switzerland (CH)
(74) Representative: Tetaz, Franck Claude Edouard

(56) References cited:
- WO-A-01/49866
- HOLLER NILS ET AL: "Two adjacent trimeric Fas ligands are required for Fas signaling and formation of a death-inducing signaling complex." MOLECULAR AND CELLULAR BIOLOGY, vol. 23, no. 4, February 2003 (2003-02), pages 1428-1440, XP002258597 ISSN: 0270-7306 (ISSN print)
- SCHNEIDER P ET AL: "APOPTOSIS INDUCED BY DEATH RECEPTORS" PHARMACEUTICA ACTA HELVETIAE, XX, XX, vol. 74, no. 2/3, March 2000 (2000-03), pages 281-286, XP001121938 ISSN: 0031-6865
- BODMER J-L ET AL: "The molecular architecture of the TNF superfamily" TIBS TRENDS IN BIOCHEMICAL SCIENCES, ELSEVIER PUBLICATION, CAMBRIDGE, EN, vol. 27, no. 1, 1 January 2002 (2002-01-01), pages 19-26, XP004332356 ISSN: 0968-0004

## Description

The present invention relates to a new use of ligands of the TNF family with reduced toxicity.

Members of the TNF receptor family and their cognate ligands have been recognized to play a major role in the control of the balance between cell proliferation and cell death in mammals. Most functions associated with the ligand/receptor system of the members of the TNF family are in relation with the control of cell proliferation, differentiation and apoptosis. Imbalance between cell death and cell proliferation can lead to various pathological conditions such as autoimmune diseases, inflammatory diseases and cancer.

Receptors of the TNF family and their ligands (cytokines) have been widely studied in the past decades and are well known in the art (Bodmer & al., TIBS, Vol. 27, No. 1, January 2002, pp. 19-27; Locksley & al., Cell 104, 487-501 (2001); Gruss and Dower, Blood, 85:3378-3404 (1995); see bibliographic parts in US application No. 20020123116, paragraphs 2-10 and US application No. 20020006391).

The receptors of the TNF receptor family are type I transmembrane proteins. They all share a typical structure of cell surface receptors with an N-terminal extracellular domain, a transmembrane and an intracellular domain. Homology identified between family members has been found mainly in the extracellular domain ("ECD") comprising repetitive cysteine-rich patterns. TNF receptor family proteins are also usually cleaved proteolytically to release soluble receptor ECDs that can function as inhibitors of the cognate cytokines (Nophar, Y. et al., EMBO J., 9:3269 (1990); and Kohno, T. et al., Proc. Natl. Acad. Sci. U.S.A., 87:8331 (1990)).

In contrast to their receptors, cytokines of the TNF family are type II transmembrane proteins, whose C-terminus is an extracellular globular head. Some cytokines of the TNF family are cleaved proteolytically at the cell surface to form a homotrimeric molecule that functions as a soluble cytokine.

Receptors of the TNF family form homotrimers when bound to their ligand (Cha & al., J. Biol. Chem. 275, 31171-31177 (2000); Hymowitz & al., Moll. Cell 4, 563-571 (1999); Mongkolsapaya & al., Nat. Struct. Biol. 6, 1048-1053 (1999)).

Several receptors of the TNF family and their cognate ligands have been identified and disclosed with a variety of different nomenclatures. The TNF Receptor Superfamily has been recently organized where the symbols for the receptor genes are based upon their relationship with the ligands:
- http://www.gene.ucl.ac.uk/nomenclature/genefamily/tnfrec2.html. Ligands are well known in the art and disclosed in various publications:
- http://www-personal.umich.edu/~ino/List/996.htm;
- http://www.gene.ucl.ac.uk/nomenclature/genefamily/tnflig.html comprising the following ligands:

| | | |
|---|---|---|
| LTA | hyphotoxin alpha (TNF superfamily, member 1) | IITNFSF1, TNFB, LT |
| TNF | tumor necrosis factor (TNF superfamily, member 2) | TNFSF2, TNFA, DIF |
| LTB | hyphotoxin beta (TNF superfamily, member 3) | TNFSF3, TNFC, p33 |
| TNFSF4 | tumor necrosis factor (ligand) superfamily, member 4 (tax-transcriptionally activated glycoprotein 1, 34kD) | OX-40L, gp34, TXGP1 |
| TNFSF5 | tumor necrosis factor (ligand) superfamily, member 5 (hyper-IgM syndrome) | CD40LG, IMD3, HIGM1, CD40L, hCD40L, TRAP, CD154, gp39 |
| TNFSF6 | tumor necrosis factor (ligand) superfamily, member 6 | FasL, APT1LG1 |
| TNFSF7 | tumor necrosis factor (ligand) superfamily, member 7 | CD70, CD27L, CD27LG |
| TNFSF8 | tumor necrosis factor (ligand) superfamily, member 8 | CD30LG |
| TNFSF9 | tumor necrosis factor (ligand) superfamily, member 9 | 4-1BB-L |
| TNFSF10 | tumor necrosis factor (ligand) superfamily, member 10 | TRAIL, Apo-2L, TL2 |
| TNFSF11 | tumor necrosis factor (ligand) superfamily, member 11 | TRANCE, RANKL, OPGL, ODF |
| TNFSF12 | tumor necrosis factor (ligand) superfamily, member 12 | TWEAK, DR3LG, APO3L |
| TNFSF13 | tumor necrosis factor (ligand) superfamily, member 13 | APRIL |
| TNFSF14 | tumor necrosis factor (ligand) superfamily, member 14 | LIGHT, LTg, HVEM-L |
| TNFSF15 | tumor necrosis factor (ligand) superfamily, member 15 | TL1.VEGI |
| TNPSF 18 | tumor necrosis factor (ligand) superfamily, member 18 | AITRL TL6 hGITRL |

Products and methods of treatment of diseases associated with disorders in the TNF family ligand/receptor interaction have been disclosed in the art, comprising administration of antibodies or ligands for the treatment of rheumatoid arthritis or Chron's disease.

Multimeric forms of such ligands have also been disclosed in the art, and more specifically multimeric forms comprising at least six soluble fractions of the ligand bounds to a multomerization tail (WO 01/49866). Such multimeric forms, also called Megaligands are agonists of the membrane-bounds cytonkines and are inducing cell death. These Megaligands have been disclosed for the treatment of various diseases where cell proliferation has to be controlled, including Neoplasia, benign and malignant (including malignant mesothelioma, metastatic ovary carcinoma, glioblastoma, metastasic colon cancer), autoimmune diseases and auto inflammatory diseases.

It was found however that due to their efficacy, some of these molecules may present high risks of toxicity depending upon their administration route. For instance, injection i.v. of a Mega-FasL (hexamer of the Fas ligand extracellular soluble fraction) to a mice, may result in almost immediate death of the mice through hepato-toxicity and liver failure.

It has been found now that injection of multimerized forms of ligands of the TNF family to specific "geographical" areas of the body could substantially reduce the toxicity of the same multimerized ligands, allowing the treatment of pathologies in these "geographical" areas of the body wherein cell proliferation has to be controlled.

The "geographical" areas of the body according to the invention are cavities of the body where organs are separated from the remaining of the body with a barrier or membrane. Once injected into the cavity, the membrane or barrier of the cavity and its cellular components will prevent the multimerized form of the ligand to substantially migrate into the general blood stream where it could affect essential organs such as the liver. Such cavities include the peritoneal cavity, the pleural cavity, the pericardial cavity, the respiratory tract (upper and lower airways), the upper digestive tract (including the mouth), the urinary tract (including bladder), the articular space and the central nervous system.

The present invention relates to the use of a multimerized form of ligands of the TNF family for the preparation of a medicament for injection into an appropriate cavity of the body, for the treatment of tumors in said cavities wherein the ligand of the TNF family is selected among Fas ligand, CD40L, TRAIL and APRIL, preferably human Fas-ligand, CD40L, TRAIL and APRIL.

Appropriate cavities are cavities of the body where the disease-associated cell proliferation has to be controlled.

Diseases or pathologies of the above cavities include all tumors in the above cavities such as primary tumors, like glioblastomas or mesothelioma (pleural and peritoneal) or secondary tumors from any cancer forms giving metastasis in the above cavities, such as ovarian metastatic cancers and colo-rectal cancers.

The multimerized forms of ligands of the TNF family comprise at least four, globular soluble extracellular fractions of the ligands of the TNF family, preferably at least five, more preferably at least six, even more preferably six globular soluble extracellular fractions of the ligands of the TNF family bounds to a multimerization moiety.

In a preferred embodiment of the invention, the multimerized form of ligand of the TNF family is an hexamer comprising six monomers, assembled together, each of the monomers comprising a polypeptide of formula (I):

H-L (I)

wherein
L represents a C-terminal ligand moiety, comprising the soluble extracellular fraction of a ligand of the TNF family selected among Fas ligand, CD40L, TRAIL and APRIL, and
H represents a N-terminal hexamerization moiety.

According to the present invention, the ligand moiety L includes the "full length" of the soluble extracellular fraction of a ligand and biologically functional fragments of the same fraction. "Biologically functional fragments" are fragments of a soluble extracellular fraction of a ligand of the TNF family conserving their ability to bind to the same receptor(s), with substantially the same affinity.

L is preferably comprises the full length extracellular soluble fraction of the above ligands.

According to an embodiment of the invention, L comprises the extracellular domain of human FAS ligand (hFasL), comprising amino acids Glu 139 to leu 281 of hFasL.

Hexamers according to the invention are either "true" hexamers, dimers of trimers or trimers of dimers. In the first case, H is a hexamerization polypeptide HP. In the latter cases, H comprises two moieties, a first moiety consisting of a dimerization polypeptide (DP) and a second moiety consisting of a trimerization polypeptide (TP).

The polypeptides according to the present invention comprise a polypeptide represented by one the following formulas (Ia), (Ib) and (Ic):
HP - L (Ia) ("true" hexamers),
DP-TP - L (Ib) (trimers of dimers), and
TP-DP - L (Ic) (dimers of trimers)
wherein L, HP, DP and TP are defined above and below.

Examples of HP, TP and DP are well known in the art and comprise isolated peptide fragments of natural hexameric, trimeric or dimeric polypeptides, the said isolated fragments being responsible for the hexamerization, dimerization or trimerization of the said natural hexamers, dimers or trimers.

Such molecules are well known in the art and comprises polypeptides of the collectin family, such as the ACRP30 or ACRP30-like proteins (WO96/39429, WO 99/10492, WO 99/59618, WO 99/59619, WO 99/64629, WO 00/26363, WO 00/48625, WO 00/63376, WO 00/63377, WO 00/73446, WO 00/73448 or WO 01/32868), apM1 (Maeda et al., Biochem. Biophys. Res. Comm. 221: 286-9, 1996), C1q (Sellar et al., Biochem. J. 274: 481-90, 1991), or C1q like proteins (WO 01/02565), which proteins comprise "collagen domains" consisting in collagen repeats Gly-Xaa-Xaa'.

Other oligomerized polypeptides are known in the art, including polypeptides with a "coiled-coil" domains (Kammerer RA, Matrix Biol 1997 Mar;15(8-9):555-65; discussion 567-8; Lombardi & al., Biopolymers 1996;40(5):495-504; http://mdl.ipc.pku.edu.cn/scop/data/scop.1.008.001.html), like the Carilage Matrix Protein (CMP) (Beck & al., 1996, J. Mol. Biol., 256, 909-923), , or polypeptides with a dimerization domain, like polypeptides with a leucine zipper or osteoprotegerin (Yamaguchi & al., 1998).

According to a specific embodiment of the invention, HP comprises the hexamerization domains of A, B or C chains of polypeptides of the C1q family.

TP are known in the art and comprise the trimerization domains (C-terminal moiety) of CMP (i.e. GeneBank 115555, amino acids 451-493) or the trimerization domain of ACRP30 and ACRP30-like molecules. According to a preferred embodiment of the present invention, TP comprises a stretch of collagen repeats.

According to the invention, a "stretch of collagen repeats" consists in a series of adjacent collagen repeats of formula (II):

- (Gly-Xaa-Xaa')ₙ - (II)

wherein Xaa and Xaa' represents independently an amino acid residue, and
n represents an integer from 10 to 40.

Xaa and Xaa' are preferably selected independently among natural amino acids such as Ala, Arg, Asn, Asp, Cys, Gln, Glu, Gly, His, lie, Leu, Lys, Met, Phe, Pro, Ser, Thr, Trp, Tyr or Val.

Xaa preferably represents independently an amino acid residue selected among Ala, Arg, Asp, Glu, Gly, His, Ile, Leu, Met, Pro or Thr, more preferably Arg, Asp, Glu, Gly, His or Thr.

Xaa' preferably represents independently an amino acid residue selected among Ala, Asn, Asp, Glu, Leu, Lys, Phe, Pro, Thr or Val, more preferably Asp, Lys, Pro or Thr.

When Xaa' represents a Pro residue, the collagen repeat Gly-Xaa-Pro is designated to be a "perfect" collagen repeat, the other collagen repeats being designated as "imperfect".

According to a preferred embodiment of the invention, the stretch of collagen repeats comprises at least 1 perfect collagen repeat, more preferably at least 5 perfect collagen repeats.

According to a preferred embodiment of the invention, n is an integer from 15 to 35, more preferably from 20 to 30, most preferably 21, 22, 23 or 24.

According to the present invention, the stretch of collagen repeat may comprise up to three "non collagen residues" inserted between two adjacent collagen repeats. These "non collagen residues" consist in 1, 2 or 3 amino acid residues, provided that when the "non collagen residue" consists in 3 amino acids residues, the first amino acid is not Gly.

According to a preferred embodiment of the invention, TP consists in an uninterrupted stretch of 22 collagen repeats. More preferably, TP consists in the stretch of 22 collagen repeats of SEQ ID NO 1, corresponding to amino acids 45 to 110 of mACRP30, as represented in SEQ ID NO 2 of WO 96/39429:

Gly Ile Pro Gly His Pro Gly His Asn Gly Thr Pro Gly Arg Asp Gly Arg Asp Gly
Thr Pro Gly Glu Lys Gly Glu Lys Gly Asp Ala Gly Leu Leu Gly Pro Lys Gly Glu
Thr Gly Asp Val Gly Met Thr Gly Ala Glu Gly Pro Arg Gly Phe Pro Gly Thr Pro
Gly Arg Lys Gly Glu Pro Gly Glu Ala

According to another preferred embodiment of the invention, TP consists in the stretch of 22 collagen repeats corresponding to amino acids 42 to 1107 of hACRP30, as represented in SEQ ID NO 7 of WO 96/39429:
DP are known in the art and comprises dimerization fragments of immunoglobulins (Fc fragments), the C-terminal dimerization domain of osteoprotegerin (Recpetor: δN-OPG; amino acids 187-401), or polypeptides sequences comprising at least 6, preferably 8 to 30 amino acids and allowing dimerization. These peptides generally comprise at least a cysteine residue allowing the formation of disulfide bonds. Other polypeptides useful as DP according to the invention are peptides designated as "leucine zippers" comprising a Leucine residue being present every seventh residue.

Examples of such peptides comprising at least a cysteine residue comprise the following peptides:

•	Val Asp Leu Glu Gly Ser Thr Ser Asn Gly Arg Gln Cys Ala Gly Ile Arg Leu

•  Glu Asp Asp Val Thr Thr Thr Glu Glu Leu Ala Pro Ala Leu Val Pro Pro Pro Lys
Gly Thr Cys Ala Gly Trp Met Ala

• Gly His Asp Gln Glu Thr Thr Thr Gln Gly Pro Gly Val Leu Leu Pro Leu Pro Lys
Gly Ala Cys Thr Gly Trp Met Ala.

The second sequence above corresponds to amino acids 17 to 44 of mACRP30 as represented in SEQ ID NO 2 of WO 96/39429, and the third sequence above corresponds to amino acids 15 to 41 of SEQ ID NO 7 of WO 96/39429.

Other peptides comprising at least one cysteine residue, can be found in amino acid sequences upstream the stretch of collagen repeats of molecules having a structure analogous to ACRP30 (ACRP30-like) as disclosed in WO 99/10492, WO 99/59618, WO 99/59619, WO 99/64629, WO 00/26363, WO 00/48625, WO 00/63376, WO 00/63377, WO 00/73446, WO 00/73448 or WO 01/32868.

Leucine zippers are well known in the art and can be found in natural proteins and eventually identified using bioinformatics tools available to the one skilled in the art (http://www.bioinf.man.ac.uk/zip/faq.shtml; http://2zip.molgen.mpg.de/; Hirst, J.D., Vieth, M., Skolnick, J. & Brooks, C.L. III, Predicting Leucine Zipper Structures from Sequence, Protein Engineering, 9, 657-662 (1996)).

The constitutive elements L, H, HP, TP and/or DP in the polypeptides of formula I, Ia, Ib or Ic, according to the invention, are assembled by peptides bonds. They may be separated by "linkers" which will not affect the functionality of the polypeptide according to the invention, its ability to form hexamers and to bind with the receptor corresponding to the ligand L. Such linkers are well known in the art of molecular biology.

The polypeptide according to the invention may also comprise peptide sequences on its N-terminus and/or C-terminus, which will not affect the functionality of the polypeptide according to the invention. These peptides may comprise affinity tags, for purification or detection of the polypeptide according to the invention. Such affinity tags are well known in the art and comprise a FLAG peptide (Hopp et al., Biotechnology 6: 1204 (1988)) or a Myc-His tag.

According to a preferred embodiment of the invention, H comprises a dimerization polypeptide (DP) and a trimerization polypeptide (TP), and is most preferably represented by the following formula:

DP-TP - L (Ib)

wherein R, DP and TP are defined above and below.

More preferably, DP and TP represent together amino acids 17 to 110 of mACRP30 as represented in SEQ ID NO 2 of WO 96/39429 or amino acids 15 to 107 of hACRP30 as represented in SEQ ID NO 7 of WO 96/39429.

A preferred embodiment of the invention the polypeptide comprises the fusion polypeptide selected among mACRP30:hFasL, mACRP30:hTRAIL, mACRP30:TNFα and mACRP30:hCD40L. Such polypeptides and their preparation are disclosed in WO 01/49866.

According to another embodiment of the invention, the hexamerization moiety comprises a Fc portion of IgG comprising amino acids 248 to 473 of gi2765420, as disclosed in WO 03/068977.

In the method according to the invention, the multimerized forms of ligands are injected in the form of a pharmaceutical composition comprising the said multimerized forms of ligands in a pharmaceutically acceptable carrier suitable for its administration by injection.

Suitable carriers, adjuvant, preservatives, etc., used prepare pharmaceutical compositions, are well-known to those in the art (Gennaro (ed.), Remington's Pharmaceutical Sciences, 19th Edition (Mack Publishing Company 1995)).

The multimerized forms of ligands according to the invention are administered to the patient in a manner such that their concentration is sufficient to bind their cognate receptors and induce cell death.

As a preferred embodiment of the present invention, the pharmaceutical composition comprises from 0.1 to 100 weight % of multimerized forms of ligands according to the invention, based on the total weight of the pharmaceutical composition, more preferably from 2.5 to 100 %. When the composition according to the invention comprises 100 % multimerized forms of ligands, it is preferably in a lyophilized form.

The multimerized form of ligands is administered from 1 to 4 times daily, at a level sufficient to achieve a total daily dose of 0.0001 to 0.2 mg/Kg/day, preferably 0.001 to 0.1 mg/kg/day.

In the method according to the invention, the multimerized forms of ligands can be used alone or in combination with one or more other mean of treatment.

By "mean of treatment" it is understood according to the invention to comprise other molecules or compositions suitable for the treatment of the same diseases, but also other means of treatment known in the art of treatment of the same diseases such as radiation therapy, chemotherapy, or eventually surgery.

Other molecules or compositions suitable for the treatment of the same diseases are well known in the art, such as any of the molecules or compositions listed under the heading "Cancerologie" in the Dictionaire Vidal (2003 ed.), in the Merk Index or in the Physician Desk Reference.

Other molecules or compositions also comprise such molecules or composition able to enhance the cell sensibility to apoptosis. Intracellular proteins involved in the control of cell death are known in the art, such as the FLIP molecules disclosed in WO 98/44104. It was shown that overexpression of FLIP in cells could inhibit apoptosis. A contrario, inhibition of FLIP or inhibition of FLIP expression could enhance the cell sensibility to apoptosis. Molecules inhibiting FLIP or FLIP expression are known in the art such as antisens molecules, disclosed in WO 98/44104 or interfering RNA molecules (RNAi or dsRNA) prepared according to methods well known in the art, such as methods disclosed in WO 00/44895, WO 02/55692 or WO 02/55693. In the method according to the invention, the multimerized forms of ligands can be injected in combination with such an antisens or dsRNA inhibiting FLIP expression.

An injection or "use" in combination with another mean according to the invention comprises the use simultaneously, separatly or sequencally of the multimerized form of the ligand and the other mean. For a simultaneous use, the multimerized form of the ligand and the other mean, preferably another molecule or composition, can be injected together in the same pharmaceutical composition, or in two separated compositions mixed together in an extemporaneous manner before injection. For a sequential administration, the multimerized form of the ligand and the other mean, preferably another molecule or composition, are in two distinct compositions. The other molecule or composition may be used according to conventional methods of administration, such as orally or by injection iv.

The present invention also concerns the use of the above multimerized forms of ligands of the TNF family as defined above, for the preparation of a medicament for injection into an appropriate cavity of the body, for the treatment of diseases wherein cell proliferation has to be controlled.

### Examples

The invention is further described in the following examples.

Except as otherwise described, all examples are carried out using standard techniques, which are well known to a person skilled in the art of molecular and/or cellular biology (i.e. T. Maniatis, E. F. Fritsch. J. Sambrook, Molecular cloning, 1982. ;M. Ausubel et al., Current Protocols in Molecular Biology, Eds., Wiley, New York, 2000).

### Example 1: Testing Mega-FasL toxicity in mice injected ip versus iv Study design:

Female Balb/c mice 8-10 weeks old will be assigned to one of the 10 treatment groups as indicated below. Mice will receive ip or iv either saline solution or Mega-FasL. Mice will be injected on Day 1 and will be bled after 2h, 6h, 24h and 48h or ALT and AST quantification. After 48h, survival will be measured and mice will be sacrificed.

| **Group** | **Number** | **Route** | **Treatment** |
|---|---|---|---|
| 1 | 6 | iv | PBS 200µl |
| 2 | 6 | iv | Mega-FasL (100µg/kg) 2µg in 200µl |
| 3 | 6 | iv | Mega-FasL (50µg/kg) 1 µg in 200µl |
| 4 | 6 | iv | Mega-FasL (25µg/kg) 0.5µg in 200µl |
| 5 | 6 | iv | Mega-FasL (12.5µg/kg) 0.25µg in 200µl |
| 6 | 6 | ip | PBS 200µl |
| 7 | 6 | ip | Mega-FasL (100µg/kg) 2µg in 200µl |
| 8 | 6 | ip | Mega-FasL (50µg/kg) 1 µg in 200µl |
| 9 | 6 | ip | Mega-FasL (25µg/kg) 0.5µg in 200µl |
| 10 | 6 | ip | Mega-FasL (12.5µg/kg) 0.25µg in 200µl |

### Liver function tests:

Bleeding mice 2h, 6h, 24h and 48 hours post injection. (Day of injection is Day 0) Collect blood (200µl) into an eppendorf tube containing 20µl of heparin (Liquémine Roche).
Centrifuge the eppendorf 3 min 5000 rpm in a desktop centrifuge
Collect the plasma into a new tube, and store it at -80°C

Results represented on the Tables below. Table 1 represents the ALT levels in mice at given times (2, 6, 24 and 48h) post injection of Mega-FasL iv (groups 1 to 5). Table 2 represents the ALT levels in mice at given times (2, 6, 24 and 48h) post injection of Mega-FasL ip (groups 6 to 10).

**Table 1**

| | **ALT U/I** | | | |
|---|---|---|---|---|
| **Group** | **2h** | **6h** | **24h** | **48h** |
| **1** | 119+/- 46 | 120+/-41 | 100+/-72 | 57+/-16 |
| **2** | 1281+/-464 | 3000 | | |
| **3** | 1085+/-267 | 5544+/-2615 | 5433+/-2366 | 1323+/-887 |
| **4** | 97+/-37 | 1450+/-1047 | 413+/-211 | 95+/-25 |
| **5** | 166+/-195 | 196+/-127 | 112+/-81 | 43+/-5 |

**Table 2**

| | **ALT U/I** | | | |
|---|---|---|---|---|
| **Group** | **2h** | **6h** | **24h** | **48h** |
| **6** | 75+/-29 | 168+/-39 | 95+/-32 | 55+/-21 |
| **7** | 97+/-21 | 268+/-130 | 409+/-227 | 96+/-44 |
| **8** | 136+/-89 | 301+/-178 | 245+/-152 | 42+/-10 |
| **9** | 96+/-55 | 183+/-100 | 194+/-81 | 56+/-8 |
| **10** | 137+/-75 | 173+/-110 | 143+/-66 | 44+/-5 |

The ALT levels at given time are more than 10 times lower after injection i.p. according to the invention compared to injection i.v. Reference ALT levels are between 35 and 51 U/1. Survival at 48h was measured and reported in the Table 3 below.

**Table 3**

| **Group** | **Number** | **Route** | **Treatment** | **Survival %** |
|---|---|---|---|---|
| 1 | 6 | iv | PBS 200µl | 100 |
| 6 | 6 | ip | PBS 200µl | 100 |
| **2** | **6** | **iv** | **Mega-FasL (100µg/kg)** | **0** |
| **7** | **6** | **ip** | **Mega-FasL (100µg/kg)** | **100** |
| **3** | **6** | **iv** | **Mega-FasL (50µg/kg)** | **30** |
| **8** | **6** | **ip** | **Mega-FasL (50µg/kg)l** | **100** |
| 4 | 6 | iv | Mega-FasL (25µg/kg) | 100 |
| 9 | 6 | ip | Mega-FasL (25µg/kg) | 100 |
| 5 | 6 | iv | Mega-FasL (12.5µg/kg)l | 100 |
| 10 | 6 | ip | Mega-FasL (12.5µg/kg) | 100 |

ALT levels in the serum represent the liver function. Increased ALT levels are specifically indicating liver damage. Therefore, the results presented on Table 2 indicate that Mega-FasL injected ip according to the present invention causes a mild liver dysfunction, while Mega-FasL injected iv causes severe liver dysfunction that can lead to the death of the animal, as seen in groups 2 and 3.

Administration of multimerized forms of ligands of the TNF family according to the invention allows the administration of higher doses of ligands, with low levels of ALT and 100% survival when 0% is observed at the same dose but injected iv.

### Example 2: Determine minimal treatment time interval for treatment of SKOV3 xenograft tumor with MegaFasL

The SKOV3 cell line is widely used model of ovarian cancer. MegaFasL triggers programmed cell death of SKOV3 in vitro (IC50 100 ng/ml). Therefore, it was of interest to test the efficacy of MegaFasL on SKOV3 tumors implanted *in vivo.*

### Study design

Mice are assigned to one of 4 treatment groups as indicated below. As pretreatment, mice of groups 1-4 receive 0.5 ml of SKOV3 cells injected I.P. on day 0. Each group is treated according to the protocol described below. Mice are sacrificed on day 22 and tumor growth is assessed after autopsy.

| **Outline** | | | | |
|---|---|---|---|---|
| **Group** | **Mice/group** | **Pre-treatment** (day 0) | **Treatment** (Days 0, 1,2, 7, 8, 9, 14, 15, 16) | **Analysis** (Day 22) |
| 1 | 6 | SKOV3 | Day 0: Day 0: MegaFasL | Autopsy |
| 2 | 6 | SKOV3 | Day 2: Day 2: MegaFasL | Autopsy |
| 3 | 6 | SKOV3 | Day 7: Day 7: MegaFasL | Autopsy |
| 4 | 6 | SKOV3 | Control PBS | Autopsy |

### Animals

Mouse strain: Athymic nude Sex: female Age: 6 wk Source: Harlan
Number of animals per group: 6
Number of groups: 4
Total mice number: 24
Animal follow-up
Body weight
Microdissection + tumor weight, histology, cryohistology
Photography of peritoneal wall.

### PROCEDURE

### Pre-treatment (tumor injection)

Groups: 1-4 (24 mice)
Cell line: SKOV3 Type: ovarian cancer Source: cell culture
Cell dose per mouse: 5 x 10⁶ cells in 500 µl PBS I.P.
Total cell number required: N/A
Injection schedule: Day 0 (26.1.2004)
*Cells:* 5 x 10⁶ SKOV3cells in PBS

### Treatment

### Group 1:MegaFasL day 0

For MegaFasL treatment, dilute a vial of 10 µg MegaFasL in 10 µl of water (stock solution at 1 µg/µl). Make up a working solution by diluting 10 µl of stock solution in 90 µl PBS (work solution at 0.1 µg/µl). Prepare treatment solution by mixing the appropriate amount of MegaFasL work solution with PBS (see treatment table). Inject 500 µl of MegaFasL treatment solution I.P. into mice. Final MegaFasL dose: **25 µg/kg**

### Group 2: MegaFasL day 2

Same as group 1, starting on day 2

### Group 3: MegaFasL day 7

Same as group 1, starting on day 7

### Group 4: PBS 500 µl per mouse

### Schedule

- Day 0: 26.1: Harvest SKOV3 cells. Resuspend at 5 x 10⁶ cells/ml. Pre-treatment:
Inject 500 µl cells I.P. into mice
Treatment: Goups 1 & 4: Inject groups with corresponding treatment
- Day 1: 27.1: Treatment: Goups 1 & 4: Inject groups with corresponding treatment
- Day 2:28.1: Treatment: Goups 1, 2 & 4 Inject groups with corresponding treatment
- Day 3: 29.1: Treatment: Goup 2 Inject group with corresponding treatment
- Day 4: 30.1: Treatment: Goup 2 Inject group with corresponding treatment
- Day 7: 2.2: Treatment: Inject groups 1-4 with corresponding treatment
- Day 8: 3.2: Treatment: Inject groups 1-4 with corresponding treatment
- Day 9: 4.2: Treatment: Inject groups 1-4 with corresponding treatment
- Day 14: 9.2: Treatment: Inject groups 1-4 with corresponding treatment
- Day 15: 10.2: Treatment: Inject groups 1-4 with corresponding treatment
- Day 16: 11.2: Treatment: Inject groups 1-4 with corresponding treatment
- Day 22: 17.2: Analysis: Mouse autopsy

Results are summarized on Table below:

| **Group** | **Nodules on Peritoneal wall** | **Solid tumor on pancreas** | **Tumor on liver stalk** |
|---|---|---|---|
| 4 (PBS control) | 3/3 | 2/3 | 3/3 |
| 1 | 1/6 | 0/6 | 0/6 |
| 2 | 0/6 | 3/6 | 1/6 |
| 3 | 0/6 | 6/6 | 5/6 |

IP administration of MegaFasL treatment prevented peritoneal tumor implantation and reduced solid tumor burden in the SKOV3 xenograft model of ovarian cancer. Tumor burden was minima after treatment with MegaFasL at Day O (Group 1). Tumor burden increased as the time interval for MegaFasL treatment was longer, although it was still reduced compared to controls at day 7.

### Example 3: Compare route and dose of SKOV3 treatment with Mega-FasL

### Study design

Mice are assigned to one of 4 treatment groups as indicated below. As pretreatment, mice of groups 1-4 receive 0.5 ml of SKOV3 cells injected I.P. on day 0. Each group is treated according to the protocol described below. Mice are sacrificed on day 22 and tumor growth is assessed after autopsy.

| **Outline** | | | | |
|---|---|---|---|---|
| **Group** | **Mice/group** | **Pre-treatment** (day 0) | **Treatment** (Days 0, 1,2, 7, 8, 9, 14, 15, 16) | **Analysis** (Day 28) |
| 1 | 6 | SKOV3 | Control PBS | Autopsy |
| 2 | 6 | SKOV3 | MegaFasL I.P. 25 µg/kg | Autopsy |
| 3 | 6 | SKOV3 | MegaFasL I.P. 5 µg/kg | Autopsy |
| 4 | 6 | SKOV3 | MegaFasL I.V. 25 µg/kg | Autopsy |

### Animals

Mouse strain: Athymic nude Sex: female Age: 6 wk Source: Harlan
Number of animals per group: 6
Number of groups: 4
Total mice number: 24

### Animal follow-up

Body weight
Macrscopy at autopsy
Microdissection, histology, cryohistology at autopsy

### 1. Pre-treatment (tumor injection)

Groups: 1-4 (24 mice)
Cell line:SKOV3 Type: ovarian cancer Source: cell culture
Cell dose per mouse: 5 x 10⁶ cells in 500 ml PBS I.P.
Total cell number required: 120 x 10⁶
Injection schedule: Day 0 (1.3.2004)
*Cells:* SKOV3 cells at 10⁷ cells/ml in PBS

### Treatment

For MegaFasL treatment, dilute a vial of 100 µg MegaFasL in 100 µl of water (stock solution at 1 µg/µl). Make up a working solution by diluting 100 µl of stock solution in 900 µl PBS (work solution at 0.1 µg/µl). Prepare treatment solution by mixing the appropriate amount of MegaFasL work solution with PBS (see treatment table). Inject 500 µl or 200 µl of MegaFasL treatment solution I.P. or I.V., respectively, into mice.

### Group 1: PBS 500 µl per mouse

### Group 2: MegaFasL day 0 / 25 µg/kg / I.P.

Same as group 1, starting on day 2

### Group 3: MegaFasL day 0 / 5 µg/kg / I.P.

Same as group 1, starting on day 7

### Group 4: MegaFasL day 0 / 25 µg/kg / I.V.

### Schedule

- Day 0: 1.3: Harvest SKOV3 cells. Resuspend at 5 x 10⁶ cells/ml
Pre-treatment: Inject 500 µl cells I.P. into mice
Treatment: Goups 1-4: Inject groups with corresponding treatment
- Day 1: 2.3: Treatment: Goups 1-4: Inject groups with corresponding treatment
- Day 2: 3.3: Treatment: Goups 1-4: Inject groups with corresponding treatment
- Day 7: 8.3: Treatment: Goup 1-4 Inject group with corresponding treatment
- Day 8: 9.3: Treatment: Goup 1-4 Inject group with corresponding treatment
- Day 9: 10.3: Treatment: Inject groups 1-4 with corresponding treatment
- Day 14: 15.3: Treatment: Inject groups 1-4 with corresponding treatment
- Day 15: 16.3: Treatment: Inject groups 1-4 with corresponding treatment
- Day 16: 17.3: Treatment: Inject groups 1-4 with corresponding treatment
- Day 31: 1.4: Analysis: Mouse autopsy

Results are summarized below
- PBS: Massive peritoneal wall tumor nodules. Pancreas, liver stalk and diaphragm: tumor deposit.
- Group 2: No peritoneal wall tumor implantation. Less (number and size) tumor deposits on pancreas, liver stalk and diaphragm. Inflammatory exudates (peritoneal adhesions)
- Group 3: Peritoneal wall tumor nodules (less than controls). Solid tumor deposits (less than controls). No inflammatory exudates.
- Group 4: Peritoneal wall tumor nodules. Solid tumor deposits. No inflammatory exudates.

MegaFasL was more efficacious at 25 µg/kg than at 5 µg/kg after IP injection. The IP route was more efficacious than the IV route at 25 µg/kg.

## Claims

1. Use of a multimerized form of ligands of the TNF family for the preparation of a medicament for injection into an appropriate cavity of the body, for the treatment of tumors in the said cavities wherein the ligand of the TNF family is selected among Fas ligand, CD40L, TRAIL and APRIL.

2. The use as claimed in claim 1, wherein the cavity is selected among the peritoneal cavity, the pleural cavity, the pericardial cavity , the respiratory tract (upper and lower airways), the upper digestive tract (including the mouth), the urinary tract (including bladder), the articular space and the central nervous system.

3. The use as claimed in claim 1 or 2, wherein the tumors in the said cavities comprise primary tumors, like glioblastomas or mesothelomia (pleural and peritoneal) or secondary tumors from any cancer forms giving metastasis in the cavities, such as ovarian metastatic cancers and colo-rectal cancers.

4. The use as claimed in one of claims 1 to 3, wherein the multimerized form of ligands of the TNF family comprises at least four, soluble extracellular fractions of the ligands of the TNF family, preferably at least five, more preferably at least six, even more preferably six soluble extracellular fractions of the ligands of the TNF family bound to a multimerization moiety.

5. The use as claimed in one of claims 1 to 4, wherein the multimerized form of ligand of the TNF family is an hexamer comprising six monomers, assembled together, each of the monomers comprising a polypeptide of formula (I):
H-L (I)
wherein
L represents a C-terminal ligand moiety, comprising the soluble extracellular fraction of a ligand of the TNF family, and
H represents an N-terminal hexamerization moiety.

6. The use as claimed in one of claims 1 to 5, wherein L is Fas ligand.

7. The use as claimed in one of claims 5 or 6, wherein H comprises amino acids 17 to 110 of mACRP30 or amino acids 15 to 107 of hACRP30.

8. The use as claimed in one of claims 1 to 4, wherein the ligand of the TNF family is human Fas ligand.

9. The use as claimed in one of claims 5 to 8, wherein Fas ligand comprises the extracellular domain of human Fas ligand (hFasL), comprising amino acids Glu 139 to leu 281 of hFasL.

## Patentansprüche

1. Verwendung einer multimerisierten Form von Liganden der TNF-Familie für die Herstellung eines Arzneimittels zur Injektion in einen geeigneten Hohlraum des Körpers für die Behandlung von Tumoren in den Hohlräumen, wobei der Ligand der TNF-Familie ausgewählt wird unter dem Fas-Ligand, CD40L, TRAIL und APRIL.

2. Verwendung nach Anspruch 1, wobei der Hohlraum aus der Bauchfellhöhle, der Pleurahöhle, der Perikardhöhle, dem Respirationstrakt (obere und untere Atemwege), dem oberen Verdauungstrakt (einschließlich des Munds), dem uropoetischen System (einschließlich der Blase), der Gelenkhöhle und dem Zentralnervensystem ausgewählt wird.

3. Verwendung nach Anspruch 1 oder 2, wobei die Tumore in den Hohlräumen primäre Tumore, wie Glioblastome oder Mesotheliome (pleurale und peritoneale), oder sekundäre Tumore ausgehend von jeglichen Krebsformen, welche in den Hohlräumen Metastasen erzeugen, wie metastasierende Eierstockkrebs-Formen und kolorektale Karzinome, umfassen.

4. Verwendung nach einem der Ansprüche 1 bis 3, wobei die multimerisierte Form von Liganden der TNF-Familie wenigstens vier lösliche extrazelluläre Fraktionen der Liganden der TNF-Familie, vorzugsweise wenigstens fünf, mehr bevorzugt wenigstens sechs, sogar noch mehr bevorzugt sechs lösliche extrazelluläre Fraktionen der Liganden der TNF-Familie gebunden an eine Multimerisierungsgruppierung umfasst.

5. Verwendung nach einem der Ansprüche 1 bis 4, wobei die multimerisierte Form von Ligand der TNF-Familie ein Hexamer ist, welches sechs Monomere umfasst, welche miteinander kombiniert sind, wobei jedes der Monomere ein Polypeptid der Formel (I) umfasst:
H-L (I),
worin
L für eine C-terminale Ligandengruppierung steht, welche die lösliche extrazelluläre Fraktion eines Liganden der TNF-Familie umfasst, und
H für eine N-terminale Hexamerisierungsgruppierung steht.

6. Verwendung nach einem der Ansprüche 1 bis 5, wobei L ein Fas-Ligand ist.

7. Verwendung nach einem der Ansprüche 5 oder 6, wobei H die Aminosäuren 17 bis 110 von mACRP30 oder die Aminosäuren 15 bis 107 von hACRP30 umfasst.

8. Verwendung nach einem der Ansprüche 1 bis 4, wobei der Ligand der TNF-Familie ein humaner Fas-Ligand ist.

9. Verwendung nach einem der Ansprüche 5 bis 8, wobei der Fas-Ligand die extrazelluläre Domäne von humanem Fas-Ligand (hFasL), umfassend die Aminosäuren Glu 139 bis Leu 281 von hFasL, umfasst.

## Revendications

1. Utilisation d'une forme multimérisée de ligands de la famille du TNF pour la préparation d'un médicament destiné à être injecté dans une cavité appropriée du corps, pour le traitement de tumeurs dans lesdites cavités, dans laquelle le ligand de la famille du TNF est choisi parmi le ligand Fas, CD40L, TRAIL et APRIL.

2. Utilisation selon la revendication 1, dans laquelle la cavité est choisie parmi la cavité péritonéale, la cavité pleurale, la cavité péricardique, l'appareil respiratoire (voies supérieures et inférieures), les voies digestives supérieures (y compris la bouche), les voies urinaires (y compris la vessie), l'espace articulaire et le système nerveux central.

3. Utilisation selon la revendication 1 ou 2, dans laquelle les tumeurs dans lesdites cavités comprennent des tumeurs primaires, telles que les glioblastomes ou les mésothéliomes (pleuraux et péritonéaux) ou des tumeurs secondaires issues de toutes formes de cancer donnant une métastase dans les cavités, telles que les cancers métastatiques ovariens et les cancers colo-rectaux.

4. Utilisation selon l'une des revendications 1 à 3, dans laquelle la forme multimérisée de ligands de la famille du TNF comprend au moins quatre fractions extracellulaires solubles des ligands de la famille du TNF, de préférence au moins cinq, de manière davantage préférée au moins six, et de manière encore plus préférée six fractions extracellulaires solubles des ligands de la famille du TNF liées à un fragment de multimérisation.

5. Utilisation selon l'une des revendications 1 à 4, dans laquelle la forme multimérisée de ligand de la famille du TNF est un hexamère comprenant six monomères, assemblés ensemble, chacun des monomères comprenant un polypeptide de formule (I) .
H-L (I)
dans laquelle
L représente une fraction C-terminale du ligand, comprenant la fraction extracellulaire soluble d'un ligand de la famille du TNF, et
H représente une fraction N-terminale d'hexamérisation.

6. Utilisation selon l'une des revendications 1 à 5, dans laquelle L est le ligand Fas.

7. Utilisation selon l'une des revendications 5 ou 6, dans laquelle H comprend les acides aminés 17 à 110 de mACRP30 ou les acides aminés 15 à 107 de hACRP30.

8. Utilisation selon l'une des revendications 1 à 4, dans laquelle le ligand de la famille du TNF est le ligand Fas humain.

9. Utilisation selon l'une des revendications 5 à 8, dans laquelle le ligand Fas comprend le domaine extracellulaire du ligand Fas humain (hFasL), comprenant les acides aminés Glu 139 à leu 281 de hFasL.
